# EUROPÄISCHE PATENTSCHRIFT

(11) **EP 0 413 217 B1**
(45) Veröffentlichungstag und Bekanntmachung des Hinweises auf die Patenterteilung: **08.06.1994**
(21) Anmeldenummer: 90114912.0
(22) Anmeldetag: 03.08.1990
(51) Int. Cl.: C07C 68/00, C07C 69/96

(54) **Verfahren zur Herstellung von Dialkylcarbonaten**
Process for the preparation of dialkyl carbonates
Procédé de préparation de carbonates de dialkyle

(30) Priorität: 12.08.1989 DE 3926710
(43) Veröffentlichungstag der Anmeldung: 20.02.1991
(73) Patentinhaber: BASF Aktiengesellschaft, 67063 Ludwigshafen (DE)
(72) Erfinder: Joerg, Klaus, Dr., D-6703 Limburgerhof (DE); Kummer, Rudolf, Dr., D-6710 Frankenthal (DE); Mueller, Franz-Josef, Dr., D-6706 Wachenheim (DE)

(56) Entgegenhaltungen:
- US-A- 4 636 576

## Beschreibung

Die vorliegende Erfindung betrifft ein Verfahren zur Herstellung von Dialkylcarbonaten der allgemeinen Formel I 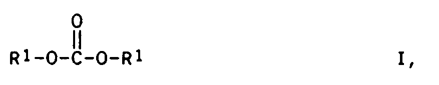
in der R¹ für eine C₁- bis C₁₀-Alkylgruppe steht, durch Umsetzung der Alkohole R¹OH mit einem Kohlenmonoxid/Sauerstoff-Gasgemisch in Gegenwart eines Kupferkatalysators und eines Cosolvens, bei erhöhter Temperatur und bei erhöhtem Druck.

Saegusa et al (J. Org. Chem. 35, 2976-2978, (1970)) beschreiben die Kupfer(methoxy)chlorid-katalysierte Herstellung von Dimethylcarbonat aus Kohlenmonoxid, Sauerstoff und Methanol.

Nach JP-45/11 129 (1970) werden zu diesem Zweck Kupfer(II)halogenide eingesetzt.

In DE-A-27 43 690 werden zur Herstellung von Dimethylcarbonat einfache Salze des einwertigen Kupfers benutzt, DE-A 21 10 194 bevorzugt hingegen die Verwendung von Kupferkomplexen mit stickstoffhaltigen Heterocyclen wie Pyridin, Dipyridyl oder Phenanthrolin. Derartige stickstoffhaltige Heterocyclen werden auch in US-A 4 761 467 in Verbindung mit Kupfer(methoxy)chlorid zur Herstellung von Kohlensäureestern verwendet, wohingegen DE-A-23 34 736 Triarylphosphinoxide, organische Phosphite, Phosphate oder Phosphonate als Komplexierungsmittel für das Kupfer bei dieser Umsetzung beansprucht. Stickstoffbasen als Komplexierungsmittel für Kupferionen bei der Herstellung von Kohlensäureestern sind Gegenstand von US-A 4 370 275.

Ein anderer Weg zur Solubilisierung der im organischen Reaktionsmedium nur schwer löslichen Kupferkatalysatoren wird in US-A 4 636 576 und US-A 4 638 076 beschritten. Anstelle der klassischen Komplexbildner werden dem Reaktionsmedium schwerflüchtige Cosolventien wie cyclische Amide, beispielsweise N-Methylpyrrolidon oder Caprolactam, bzw. Phosphorsäureamide, beispielsweise Hexamethylphosphorsäuretriamid oder Tri(pentamethylen)phosphorsäuretriamid zugesetzt.

Die Vielzahl der geschilderten Vorgehensweise zur Herstellung von Dialkylcarbonaten zeigt, daß die jeweils propagierten Lösungen noch zahlreiche Mängel wie unbefriedigende Raum-Zeit-Ausbeuten, Probleme bei der Aufarbeitung des Reaktionsgemisches, Korrosionsprobleme und Probleme im Zusammenhang mit der Verwendung toxischer Komplexbildner oder Cosolventien haben. Der vorliegenden Erfindung lag daher die Aufgabe zugrunde, ein Verfahren zur Herstellung von Dialkylcarbonaten zu finden, das nicht mit den Nachteilen der bisherigen Verfahren behaftet ist.

Dementsprechend wurde ein Verfahren zur Herstellung von Dialkylcarbonaten der allgemeinen Formel I 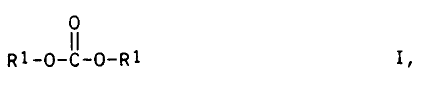
in der R¹ für eine C₁- bis C₁₀-Alkylgruppe steht, durch Umsetzung der Alkohole R¹OH mit einem Kohlenmonoxid/Sauerstoff-Gasgemisch in Gegenwart eines Kupferkatalysators und eines Cosolvens, bei erhöhter Temperatur und bei erhöhtem Druck, gefunden, das dadurch gekennzeichnet ist, daß man als Cosolvens einen cyclischen Harnstoff der allgemeinen Formel II 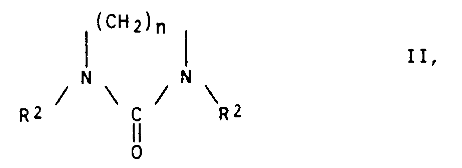
in der n die Zahl 2, 3 oder 4 bedeutet, einsetzt und R² für Wasserstoff oder eine C₁- bis C₄-Alkylgruppe steht, verwendet.

Als Cosolventien werden im erfindungsgemäßen Verfahren cyclische Harnstoffe der allgemeinen Formel II verwendet, in der n für eine der Zahlen 2, 3 oder 4 steht und in der R² Wasserstoff oder die Methyl-, Ethyl-, Propyl- oder Butylgruppe steht. Die Reste R² können gleich oder verschieden, geradkettig oder verzweigt sein. Vorzugsweise werden aber cyclische Harnstoffe verwendet, in denen die Reste R² gleich und geradkettige Alkylgruppen sind. Besonders bevorzugte Cosolventien sind Dimethylethylenharnstoff (DMEU) der Formel III 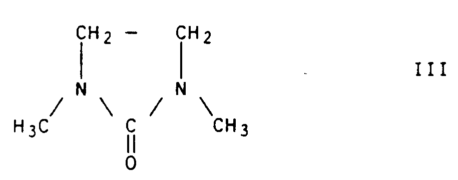
und Dimethylpropylenharnstoff (DMPU) der Formel IV. 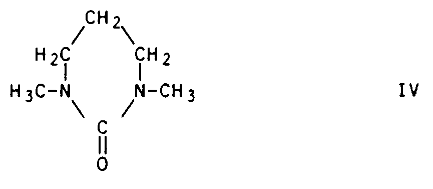
Selbstverständlich können auch Mischungen verschiedener cyclischer Harnstoffe als Cosolventien verwendet werden.

Zur Herstellung der Dialkylcarbonate I, welche formal gemäß Reaktionsgleichung (1) abläuft, 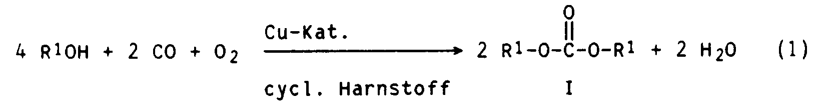
wird das umzusetzende Alkanol zusammen mit dem cyclischen Harnstoff und dem Kupferkatalysator im Reaktor vorgelegt. Dabei beträgt im allgemeinen das Molverhältnis Alkanol : cyclischem Harnstoff : Kupfersalz 1 : 0,1-1 : 0,01 - 0,1, vorteilhaft 1 : 0,2 - 0,6 : 0,03 - 0,07 und insbesondere 1 : 0,4 - 0,5 : 0,04 - 0,06.

Im allgemeinen werden gemäß dem erfindungsgemäßen Verfahren C₁- bis C₁₀-Alkanole zu den entsprechenden Dialkylcarbonaten umgesetzt. Die Umsetzung höherer Homologer ist ebenfalls möglich, wirtschaftlich jedoch ohne Bedeutung. Die umzusetzenden Alkohole können geradkettig oder verzweigt sein. Vorzugsweise werden nach dem erfindungsgemäßen Verfahren C₁- bis C₄-Alkanole zu Dialkylcarbonaten umgesetzt, besonders bevorzugt ist die Herstellung von Dimethyl- und Diethylcarbonat.

Als Kupferkatalysatoren finden im erfindungsgemäßen Verfahren im allgemeinen einfache Kupfersalze wie Kupferhalogenide, bevorzugt Kupfer(I)halogenide, wie Kupfer(I)chlorid, Kupfer(I)bromid oder Kupfer(I)iodid, Kupfersulfat oder Kupfer(alkoxy)halogenide, wie Kupfer(methoxy)chlorid, Kupfer(ethoxy)chlorid, Kupfer(propoxy)chlorid, Kupfer(butoxy)chlorid oder Kupfer(methoxy)bromid Verwendung. Bei Verwendung von Kupfer(alkoxy)halogeniden als Katalysator ist es vorteilhaft, eine dem umzusetzenden Alkanol entsprechende Alkoxykomponente zu wählen, da anderenfalls gemischte Dialkylcarbonate entstehen, was im allgemeinen nicht gewünscht wird.

Da es sich bei der Umsetzung gemäß Gleichung (1) um eine von den Kupfersalzen katalysierte Redoxreaktion handelt, bildet sich im Zuge der Reaktion in der Reaktionsmischung ein Gleichgewicht aus Kupfer(I)- und Kupfer(II)salzen aus, das unabhängig davon ist, ob man beispielsweise von Kupfer(I)halogeniden oder Kupfer(alkoxy)halogeniden als Einsatzstoffen ausgeht.

Die Reaktion wird in Gang gesetzt, indem man ein Kohlenmonoxid/Sauerstoff-Gasgemisch in die Reaktionsmischung einleitet. Die Zusammensetzung dieses Gasgemisches kann in weiten Grenzen beliebig gewählt werden, zweckmäßigerweise arbeitet man jedoch mit Gasgemischen, deren Sauerstoffgehalt so gering gehalten ist, daß sich keine explosionsfähigen Gasgemische bilden können, und deren Sauerstoffgehalt genügend groß ist, um dennoch einen zufriedenstellenden Umsatz und damit eine gute Raum-Zeit-Ausbeute zu gewährleisten. Im allgemeinen wird ein auf 1 normiertes CO/O₂-Molverhältnis von 1 : 0,05 - 0,15, vorzugsweise 1 : 0,05 - 0,10, insbesondere von 1 : 0,06 - 0,08 angewandt.

Die Reaktionsgase - Kohlenmonoxid und Sauerstoff - werden in die im Reaktor befindliche Flüssigkeit eingeleitet. Zur besseren Dispergierung der Reaktionsgase kann die flüssige Reaktionsmischung im Reaktor zusätzlich mechanisch gerührt werden. Als Reaktortyp wird zweckmäßigerweise ein Blasenreaktor gewählt, welcher beheizt werden kann und gewünschtenfalls zusätzlich mit einer Rührvorrichtung versehen ist.

Die Umsetzung wird zweckmäßigerweise unter erhöhtem Druck, im allgemeinen bei einem Druck von 1 bis 100, vorzugsweise von 10 bis 50 und besonders bevorzugt von 20 bis 30 bar durchgeführt. Die Reaktionstemperatur beträgt im allgemeinen 70 bis 150, vorteilhaft 80 bis 130, insbesondere 90 bis 120°C.

Die Umsetzung kann kontinuierlich oder diskontinuierlich ausgeführt werden. Bei der kontinuierlichen Betriebsweise werden die Reaktionsgase so durch das Reaktionsmedium geleitet, daß das nicht reagierte Kohlenmonoxid/Sauerstoff-Abgas, das gebildete Dialkylcarbonat zusammen mit dem Reaktionswasser und zusammen mit der zur Bildung eines niedrigsiedenden, ternären Azeotrops erforderlichen Menge an Alkanol laufend, d. h. wie es sich bildet, aus der Reaktionsmischung strippt. Dieser Effekt wird im allgemeinen dadurch erzielt, indem man Gasströme von 10 bis 50, vorteilhaft von 15 bis 35, insbesondere von 20 bis 30 Nl CO/O₂-Gasgemisch pro g im Reaktor als Kupferkatalysator vorhandenen Kupfers durch die flüssige Reaktionsmischung leitet. Der Kupferkatalysator und das Cosolvens verbleiben unter diesen Bedingungen im Reaktor.

Der gasförmige Reaktionsaustrag wird anschließend in einem Kühler in seine bei Raumtemperatur gasförmigen - Kohlenmonoxid und Sauerstoff - und flüssigen - Dialkylcarbonat, Reaktionswasser und Alkanol - Bestandteile zerlegt. Das Reaktionsabgas kann zweckmäßigerweise nach Ergänzung seines Kohlenmonoxid- und Sauerstoffgehaltes nach Maßgabe des Verbrauchs dieser Gase wieder in die Umsetzung zurückgeführt werden. Die dem Abscheider entnommenen flüssigen Bestandteile werden destilliert, das Dialkylcarbonat isoliert und das Alkanol gewünschtenfalls wieder in den Reaktor geleitet.

Im chargenweisen Betrieb können nach dem erfindungsgemäßen Verfahren Dimethylcarbonat-Ausbeuten in Höhe von 80 - 90 % erzielt werden.

### Beispiele

### Beispiel 1

105 g wasserfreies Methanol, 14,0 g CuOMeCl (0,11 mol) und 40 g DMPU (Dimethylpropylenharnstoff, Sdp. 246°C) wurden in einem 0,3 l korrosionsbeständigen Autoklaven 30 Minuten lang bei 90°C und 35 bar CO-Druck umgesetzt. Nach Entspannen und Abkühlen der Reaktionsmischung enthielt der Austrag laut gaschromatographischer Analyse bis zu 90 % Dimethylcarbonat (bez. auf eingesetztes Cu-Salz). Die Selektivität betrug 99 %.

### Beispiel 2

105 g wasserfreies Methanol, 10,5 g CuCl (0,11 mol) und 40 g DMEU (Dimethylethylenharnstoff, Sdp. 225°C) wurden zuerst 15 Minuten lang bei 90°C und 8 bar O₂-Druck umgesetzt. Anschließend wurde O₂ durch CO ersetzt und 30 Minuten lang bei 35 bar das Methanol zu Dimethylcarbonat (DMC) umgewandelt. Die Ausbeute an DMC beträgt laut GC-Analyse 76 % (bez. auf eingesetztes Cu-Salz).

## Patentansprüche

1. Verfahren zur Herstellung von Dialkylcarbonaten der allgemeinen Formel I 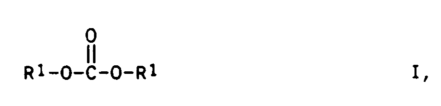 in der R¹ für eine C₁- bis C₁₀-Alkylgruppe steht, durch Umsetzung der Alkohole R¹OH mit einem Kohlenmonoxid/Sauerstoff-Gasgemisch in Gegenwart eines Kupferkatalysators und eines Cosolvens, bei erhöhter Temperatur und bei erhöhtem Druck, dadurch gekennzeichnet, daß man als Cosolvens einen cyclischen Harnstoff der allgemeinen Formel II 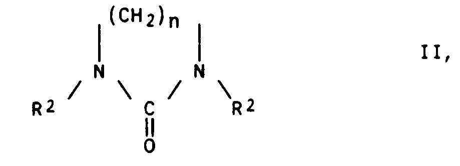 in der n die Zahl 2, 3 oder 4 bedeutet und R² für Wasserstoff oder eine C₁- bis C₄-Alkylgruppe steht, verwendet.

2. Verfahren nach Anspruch 1, dadurch gekennzeichnet, daß man als Kupferkatalysator Kupfer(methoxy)chlorid oder -bromid verwendet.

3. Verfahren nach Anspruch 1, dadurch gekennzeichnet, daß man als Kupferkatalysator Kupfer(I)chlorid oder -bromid verwendet.

4. Verfahren gemäß Anspruch 1, dadurch gekennzeichnet, daß man als Cosolvens Dimethylethylenharnstoff und/oder Dimethylpropylenharnstoff verwendet.

## Claims

1. A process for preparing a dialkyl carbonate of the general formula I 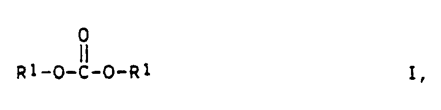 where R¹ is a C₁- to C₁₀-alkyl group, by reacting the alcohol R¹OH with a carbon monoxide/oxygen gas mixture in the presence of a copper catalyst and of a cosolvent, at elevated temperature and at elevated pressure, which comprises using as cosolvent a cyclic urea of the general formula II 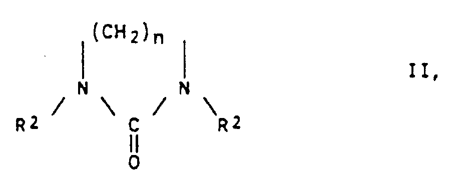 where n is the number 2, 3 or 4 and R² is hydrogen or a C₁- to C₄-alkyl group.

2. The process as claimed in claim 1, wherein the copper catalyst used is copper (methoxy)chloride or (methoxy)bromide.

3. The process as claimed in claim 1, wherein the copper catalyst used is copper(I) chloride or bromide.

4. The process as claimed in claim 1, wherein the cosolvent used is dimethylethyleneurea and/or dimethylpropyleneurea.

## Revendications

1. Procédé de préparation de carbonates de dialkyle de formule générale I 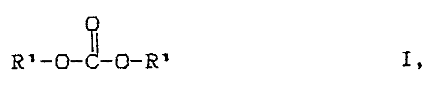 dans laquelle R¹ est mis pour un groupe alkyle en C₁-C₁₀, par réaction des alcools R¹OH avec un mélange gazeux monoxyde de carbone/oxygène en présence d'un catalyseur au cuivre et d'un co-solvant, à température élevée et sous une pression élevée, caractérisé en ce que l'on utilise comme co-solvant une urée cyclique de formule générale II 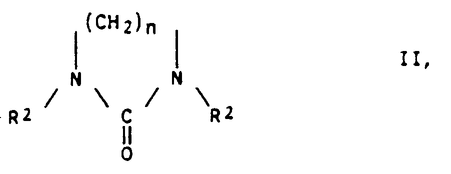 dans laquelle n signifie 2, 3 ou 4 et R² est mis pour un atome d'hydrogène ou un groupe alkyle en C₁-C₄.

2. Procédé selon la revendication 1, caractérisé en ce que l'on utilise comme catalyseur au cuivre du (méthoxy)chlorure ou (méthoxy)bromure de cuivre.

3. Procédé selon la revendication 1, caractérisé en ce que l'on utilise comme catalyseur au cuivre du chlorure ou du bromure de cuivre (I).

4. Procédé selon la revendication 1, caractérisé en ce que l'on utilise comme co-solvant de la diméthyléthyléne-urée et/ou de la diméthylpropyléneurée.
